# EUROPEAN PATENT SPECIFICATION

(11) **EP 4 096 579 B1**
(45) Date of publication and mention of the grant of the patent: **12.06.2024**
(21) Application number: 21717742.7
(22) Date of filing: 23.03.2021
(51) Int. Cl.: A61F 2/24, A61F 2/95

(54) **DELIVERY APPARATUS FOR AN IMPLANTABLE MEDICAL DEVICE**
FREISETZUNGSVORRICHTUNG FÜR EIN IMPLANTIERBARES MEDIZINPRODUKT
APPAREIL D'ADMINISTRATION POUR DISPOSITIF MÉDICAL POUVANT ÊTRE IMPLANTÉ

(30) Priority: 26.03.2020 US 202063000225 P
(43) Date of publication of application: 07.12.2022
(73) Proprietor: Edwards Lifesciences Corporation, Irvine, CA 92614-5688 (US)
(72) Inventor: PINEDJIAN, Raffi, Sempad, Irvine, CA 92614 (US); GAFFNEY, Leah Paige, Irvine, CA 92614 (US)
(74) Representative: Venner, Julia Ann
(86) International application number: PCT/US2021/023696
(87) International publication number: WO 2021/195090

(56) References cited:
- JP-A- 2015 178 749
- US-A- 4 363 186
- US-A1- 2014 343 670
- US-A1- 2020 015 968
- US-B1- 6 221 409

## Description

### CROSS REFERENCE TO RELATED APPLICATIONS

This application claims the benefit of U.S. Provisional Application No. 63/000,225, filed March 26, 2020.

### FIELD

The present disclosure concerns embodiments of a delivery apparatus for an implantable medical device, such as a prosthetic valve.

### BACKGROUND

Delivery devices, such as endovascular delivery devices, are used in various procedures to deliver prosthetic medical devices to locations inside the body that are not readily accessible by surgery or where access without surgery is desirable. Access to a target location inside the body is achieved by a medical specialist inserting and guiding the delivery device through a pathway or lumen in the body, including, but not limited to, a blood vessel, an esophagus, a trachea, any portion of the gastrointestinal tract, a lymphatic vessel, to name a few. The prosthetic medical device may include expandable valves or instruments (e.g., stents). In one specific example, an expandable prosthetic heart valve can be mounted in a crimped state on a distal end of the delivery device and then deployed from a capsule of the delivery device at the implantation site so that the prosthetic valve can self-expand to its functional size.

In some embodiments, for ease of use, the delivery device can be motorized via the inclusion of a motor in a handle portion of the device. During delivery of the prosthetic valve, the medical specialist operates the motor to retract the capsule and deploy the valve. Although rare, electronic components can malfunction. If the motor fails to operate during valve deployment, the valve delivery procedure can be compromised.

US 2020/0015968 describes a system for delivering an implantable medical device including an outer sheath drive assembly and an actuation shaft drive assembly. The outer sheath drive assembly is configured to cause an outer sheath to translate relative to a handle and includes an outer sheath drive motor. The actuation shaft drive assembly is configured to cause an actuation shaft to translate relative to the handle and includes an actuation shaft drive motor. The outer sheath drive motor and/or the actuation shaft drive motor may be removed from the system, and the outer sheath drive assembly and/or the actuation shaft drive assembly may instead be manually driven.

### SUMMARY

The presently claimed invention concerns a medical apparatus according to independent claim 1 and a delivery apparatus according to independent claim 5. Particular embodiments of these aspects are set out in the dependent claims. Any subject matter contained herein that does not fall within the scope of the appended claims is considered as being useful for understanding the invention.

Disclosed herein are embodiments of an improved delivery apparatus for an implantable medical device (e.g., a prosthetic heart valve), as well as related methods, not covered by the presently claimed invention, for use of such an apparatus in implanting an implantable medical device in a subject. The delivery apparatus is operated via a motor and comprises a manual deployment tool which can serve as a back-up or "bail out" solution in the event of motor malfunction.

Disclosed herein is a delivery apparatus that comprises a handle portion which is used by a medical specialist to operate the apparatus. The delivery apparatus can have a shaft extending from the handle portion; a delivery capsule configured to house the medical device in a radially compressed state for delivery into a subject; and a rotatable component disposed in the handle portion and operatively coupled to the delivery capsule to produce axial movement of the delivery capsule relative to the shaft upon rotation of the rotatable component. The delivery apparatus can have a motor disposed in the handle portion and operatively coupled to the rotatable component to produce rotation of the rotatable component and corresponding axial movement of the delivery capsule.

The delivery apparatus further includes a pull cord, configured to produce rotation of the rotatable component and corresponding axial movement of the delivery capsule when a manual pulling force is applied to the pull cord to pull the pull cord relative to the rotatable component.

For example, in the event of motor function failure during a valve deployment procedure, the user can operate the manual deployment tool to manually complete the deployment procedure.

Disclosed herein is a delivery apparatus for delivering an expandable, implantable medical device which comprises a handle portion; a shaft extending from the handle portion; a delivery capsule configured to house the medical device in a radially compressed state for delivery into a subject; a rotatable component disposed in the handle portion and operatively coupled to the delivery capsule to produce axial movement of the delivery capsule relative to the shaft upon rotation of the rotatable component, wherein the rotatable component comprises a plurality of circumferentially arrayed gear teeth; a motor disposed in the handle portion and operatively coupled to the rotatable component to produce rotation of the rotatable component and corresponding axial movement of the delivery capsule; and a manual deployment tool comprising a plurality of drive teeth configured to engage the gear teeth of the rotatable component, wherein the deployment tool is manually movable along an axis extending through the handle portion to produce rotation of the rotatable component and corresponding axial movement of the delivery capsule.

Also disclosed herein is a method, not covered by the presently claimed invention, of implanting a medical device in a subject which comprises inserting the medical device into the subject's vasculature (or equivalent thereof) with a delivery apparatus, wherein the medical device is retained in a radially compressed state within a delivery capsule of the delivery apparatus. The delivery apparatus comprises a handle portion and a rotatable component housed in the handle portion. The method further comprises pulling a pull cord through the handle portion to produce rotation of the rotatable component, which in turn produces axial movement of the delivery capsule relative to the medical device to deploy the medical device from the delivery capsule and allow the medical device to radially expand from the radially compressed state to a radially expanded state.

Also disclosed herein is a method, not covered by the presently claimed invention, of implanting a medical device in a subject which comprises inserting the medical device into the subject's vasculature (or equivalent thereof) with a delivery apparatus, wherein the medical device is retained in a radially compressed state within a delivery capsule of the delivery apparatus, wherein the delivery apparatus comprises a handle portion and a rotatable component housed in the handle portion. The method further includes operating an electric switch to actuate a motor operatively coupled to the rotatable component. If the motor fails to produce movement of the delivery capsule, the pull cord is pulled through the handle portion to produce rotation of the rotatable component, which in turn produces axial movement of the delivery capsule in a first direction relative to the medical device to deploy the medical device from the delivery capsule and allow the medical device to radially expand from the radially compressed state to a radially expanded state. Alternatively, if the motor fails when the medical device is partially deployed from the delivery capsule, the cord is pulled through the handle portion to produce rotation of the rotatable component, which in turn produces axial movement of the delivery capsule in a second direction relative to the medical device to recapture the partially deployed medical device.

Disclosed herein is a delivery apparatus for an expandable, implantable medical device which comprises a handle portion, a delivery capsule configured to house the medical device in a radially compressed state for delivery into a subject, a rotatable component disposed in the handle portion and operatively coupled to the delivery capsule to produce axial movement of the delivery capsule relative to the handle portion upon rotation of the rotatable component, and a pull cord configured to produce rotation of the rotatable component and corresponding axial movement of the delivery capsule when a manual pulling force is applied to the pull cord to pull the pull cord relative to the rotatable component.

Disclosed herein is a medical apparatus for insertion into a subject which comprises a handle portion, a moveable component configured to be inserted into the subject, a rotatable component disposed in the handle portion and operatively coupled to the moveable component to produce axial movement of the moveable component relative to the handle portion upon rotation of the rotatable component, and a pull cord configured to produce rotation of the rotatable component and corresponding axial movement of the moveable component when a manual pulling force is applied to the pull cord to pull the pull cord relative to the rotatable component.

Also disclosed herein is a method, not covered by the presently claimed invention, of using a medical apparatus which comprises inserting a moveable component of the medical apparatus into a subject, wherein the medical apparatus comprises a handle portion and a rotatable component in the handle portion; and pulling a pull cord through the handle portion to produce rotation of the rotatable component and corresponding axial movement of the moveable component.

The foregoing and other objects, features, and advantages of the disclosed technology will become more apparent from the following detailed description, which proceeds with reference to the accompanying figures.

### BRIEF DESCRIPTION OF THE DRAWINGS

FIG. 1 is a side elevation view of an exemplary embodiment of an implantable prosthetic heart valve that can be implanted using any of the delivery apparatuses disclosed herein.
FIG. 2 is a side elevation view of an exemplary embodiment of a delivery apparatus for delivering the prosthetic heart valve of FIG. 1.
FIG. 3 is a side cross-sectional view of the distal end portion of the delivery apparatus of FIG. 2 showing the prosthetic valve housed in a compressed state within a delivery capsule.
FIG. 4 is a side elevation view of the distal end portion of the delivery apparatus of FIG. 2 shown with the capsule of the delivery apparatus advanced over a portion of the prosthetic heart valve frame.
FIG. 5 is a side elevation view of the handle portion of the delivery apparatus of FIG. 2 showing an opening for receiving a manual deployment tool for manually operating the delivery apparatus.
FIG. 6 is a side view of the handle portion of FIG. 5 with the housing of the handle portion shown in cross-section.
FIG. 7 is a top perspective, exploded view of the handle portion of the delivery apparatus of FIG. 2 and an embodiment of a manual deployment tool configured to enable manual deployment or recapture of a prosthetic valve from the delivery apparatus.
FIG. 8 is a side cross-sectional view of the handle portion of the delivery apparatus of FIG. 2.
FIGS. 9A-9B are cross-sectional views of the handle portion of the delivery apparatus of FIG. 2 showing the engagement of the manual deployment tool, configured as a pull cord, with a drive gear of the handle portion.
FIG. 10 is a side elevation view of a delivery apparatus, according to another embodiment.
FIG. 11 is a side view of another embodiment of a manual deployment tool configured to interface with a rotatable component of a handle portion of a delivery apparatus and enable manual deployment or recapture of a prosthetic valve from the delivery apparatus, the manual deployment tool having a straight configuration.
FIG. 12 is a side view of another embodiment of a manual deployment tool configured to interface with a rotatable component of a handle portion of a delivery apparatus and enable manual deployment or recapture of a prosthetic valve from the delivery apparatus, the manual deployment tool having a coiled configuration.

### DETAILED DESCRIPTION

### General Considerations

For purposes of this description, certain aspects, advantages, and novel features of the embodiments of this disclosure are described herein. The described methods, systems, and apparatus should not be construed as limiting in any way. Instead, the present disclosure is directed toward all novel and non-obvious features and aspects of the various disclosed embodiments, alone and in various combinations and sub-combinations with one another. The disclosed methods, systems, and apparatus are not limited to any specific aspect, feature, or combination thereof, nor do the disclosed methods, systems, and apparatus require that any one or more specific advantages be present, or problems be solved.

Although the operations of some of the disclosed methods are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods, systems, and apparatus can be used in conjunction with other systems, methods, and apparatus.

As used herein, the terms "a," "an," and "at least one" encompass one or more of the specified element. That is, if two of a particular element are present, one of these elements is also present and thus "an" element is present. The terms "a plurality of" and "plural" mean two or more of the specified element.

As used herein, the term "and/or" used between the last two of a list of elements means any one or more of the listed elements. For example, the phrase "A, B, and/or C" means "A," "B," "C," "A and B," "A and C," "B and C," or "A, B, and C."

As used herein, the term "coupled" generally means physically coupled or linked and does not exclude the presence of intermediate elements between the coupled items absent specific contrary language.

Directions and other relative references (e.g., inner, outer, upper, lower, etc.) may be used to facilitate discussion of the drawings and principles herein, but are not intended to be limiting. For example, certain terms may be used such as "inside," "outside,", "top," "down," "interior," "exterior," and the like. Such terms are used, where applicable, to provide some clarity of description when dealing with relative relationships, particularly with respect to the illustrated embodiments. Such terms are not, however, intended to imply absolute relationships, positions, and/or orientations. For example, with respect to an object, an "upper" part can become a "lower" part simply by turning the object over. Nevertheless, it is still the same part and the object remains the same. As used herein, "and/or" means "and" or "or," as well as "and" and "or."

In the context of the present application, the terms "lower" and "upper" are used interchangeably with the term's "inflow" and "outflow", respectively. Thus, for example, the lower end of the valve is its inflow end and the upper end of the valve is its outflow end.

As used herein, with reference to the prosthetic heart valve and the delivery apparatus, "proximal" refers to a position, direction, or portion of a component that is closer to the user and/or a handle of the delivery apparatus that is outside the subject, while "distal" refers to a position, direction, or portion of a component that is further away from the user and/or the handle of the delivery apparatus and closer to the implantation site. The terms "longitudinal" and "axial" refer to an axis extending in the proximal and distal directions, unless otherwise expressly defined. Further, the term "radial" refers to a direction that is arranged perpendicular to the axis and points along a radius from a center of an object (where the axis is positioned at the center, such as the longitudinal axis of the prosthetic valve).

### Examples of the Disclosed Technology

Described herein are examples of a motorized delivery apparatus that can be used to deliver an implantable, expandable medical device, such as a prosthetic heart valve. The disclosed delivery apparatus comprises a manual deployment tool that can be used to operate the apparatus in the event of motor failure, thereby improving a physician's ability to complete deployment and implantation of the medical device during a procedure. In some representative embodiments, the manual deployment tool is configured as a pull cord that is pulled through an opening in a handle portion of the delivery apparatus to manually operate the apparatus.

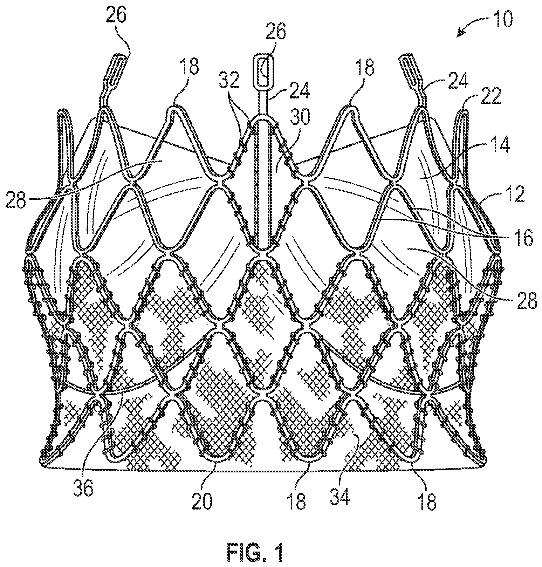

In some embodiments, the delivery apparatus is configured to deliver and implant a prosthetic heart valve, such as the example prosthetic heart valve of FIG. 1, at a selected implantation site within a subject (e.g., within the native aortic valve, mitral valve, tricuspid valve or pulmonary valve). In addition to prosthetic heart valves, disclosed delivery apparatuses can be adapted to deliver and implant other types of prosthetic valves (e.g., venous valves) and various other types of prosthetic devices, such as stents, grafts, docking devices for prosthetic heart valves, heart valve repair devices (e.g., leaflet clips), embolic coils, and the like; to position imaging devices and/or components thereof, including ultrasound transducers; and to position energy sources, for example, devices for performing lithotripsy, RF sources, ultrasound emitters, electromagnetic sources, laser sources, thermal sources, and the like.

In various embodiments described herein, delivery apparatuses and methods may be deployed or performed within a subject. Subjects include (but are not limited to) medical patients, veterinary patients, animal models, cadavers, and simulators of the cardiac and vasculature system (e.g., anthropomorphic phantoms and explant tissue). Accordingly, various embodiments are directed to methods for medical procedures, practice of medical procedures, and/or training of medical procedures. Simulators may include a simulation of whole or partial vasculature system, a whole or partial heart, and/or whole or partial components of the vasculature system (e.g., whole or partial ascending aorta). References to native tissue (e.g., native heart valve) refer to preexisting structures within the subject, such as (for example) native tissue of a patient or a component of a simulator.

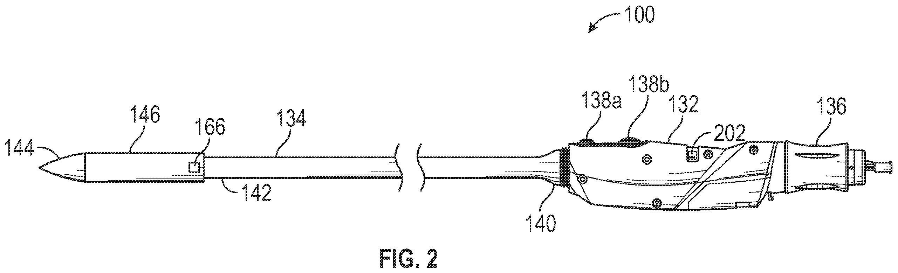

FIG. 1 shows a prosthetic heart valve 10, according to one embodiment, that can be implanted with a delivery apparatus 100 of FIG. 2. In some embodiments, the prosthetic heart valve is a self-expanding valve that is delivered in a radially compressed state to a deployment site via the delivery apparatus 100. When advanced from a delivery capsule at the distal end of the delivery apparatus (FIG. 2), the prosthetic valve can radially self-expand to its functional size.

The prosthetic heart valve 10 comprises a stent, or frame 12 and a valvular structure 14 (e.g., leaflets or a flap valve) supported by the frame. The frame 12 can have a plurality of interconnected struts 16 arranged in a lattice-like pattern and forming a plurality of apices 18 at the inflow and outflow ends 20, 22, respectively, of the frame 12

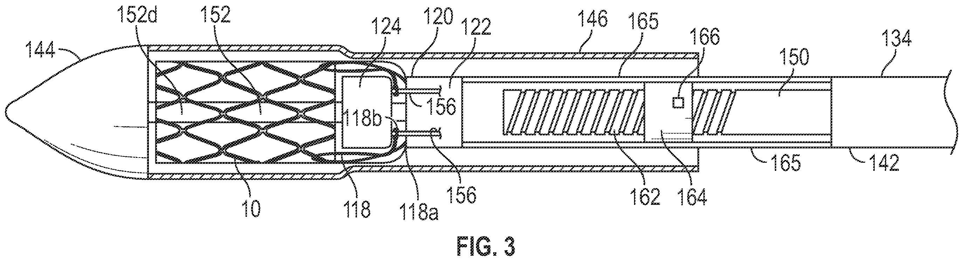

The frame 12 can include a plurality of angularly-spaced posts 24 extending from respective apices 18 at the outflow end of the frame 12. The frame 12 in the illustrated embodiment includes three such posts 24, although a greater or fewer number of posts can be used. In one implementation, the frame 12 can have posts extending from all the apices 18 at the outflow end of the frame. Each post 24 can have an eyelet or aperture 26, which can be used to form a releasable connection with the delivery apparatus 100, such as via the use of one or more cords or tethers 118 (see FIG. 3), as further described below.

In some embodiments, the frame 12 can be without posts 24 and apertures 26 can be formed in the apices 18 at the outflow end of the frame. In the embodiment shown at FIG. 3, the apertures are formed at the outflow end of the frame so that when loaded within the delivery apparatus 100, a releasable connection can be formed between a cord manifold 120 and the outflow end of the frame 12 via cords 118, as further described below. This arrangement facilitates delivery of the prosthetic valve 10 to the native aortic valve using a retrograde delivery approach whereby the delivery apparatus 100 is advanced through a femoral artery and the aorta to access the native aortic valve.

In other embodiments, the apertures 26 (whether formed in posts 24 or in the apices 18) can be formed at the inlet (or inflow) end 20 of the frame 12 where other delivery apparatus configurations or other delivery techniques require apertures at the inlet end of the frame, such as a transapical delivery approach. In still further embodiments, the delivery apparatus 100 can include the cord manifold 120 positioned distal to the prosthetic valve when loaded within the delivery apparatus, with the cord manifold coupled to the inlet (or inflow) end 20 of the frame.

In particular embodiments, the prosthetic heart valve 10 is a self-expandable heart valve wherein the frame 12 is a made of a super-elastic, self-expanding material (e.g., a nickel titanium alloy such as Nitinol) as is known in the art. When used with the delivery apparatus 100 (FIG. 2), the prosthetic valve 10 can self-expand from a radially compressed state to a radially expanded state when advanced from a delivery capsule (e.g., a delivery sheath) of the delivery apparatus.

In other embodiments, the frame 12 can be made of any of various suitable plastically-expandable materials (e.g., stainless steel, cobalt-chromium alloy, etc.) and the prosthetic heart valve can be expanded from a radially compressed state to a radially expanded state by inflating a balloon of the delivery apparatus or by actuating other expansion means of the delivery apparatus and produces radial expansion of the prosthetic valve.

The valvular structure 14 can comprise a plurality of leaflets 28. The valvular structure typically comprises three leaflets 28 arranged in a tricuspid arrangement, although a greater or fewer number of leaflets 28 can be used. The leaflets 28 can be made of any various suitable materials, including natural tissue (e.g., bovine pericardium or pericardium from other sources) or synthetic materials (e.g., polyurethane). Adjacent side portions at the outflow edges (the upper edges in the drawings) of adjacent leaflets can be secured to each other to form commissures 30 of the valvular structure, which can be secured to the frame with sutures 32.

The prosthetic valve 10 can further include an inner skirt 34 mounted on the inside of the frame 12. The skirt 34 helps establish a seal with the surrounding tissue after implantation. The skirt 34 can also be used to mount portions of the leaflets 28 to the frame 12. For example, in the illustrated embodiment, the inflow edges of the leaflets (the lower edges in the drawings) can be sutured to the skirt 34 along suture line 36. The skirt 34 can be connected directly to the frame 12, such as with sutures. Although not shown, the prosthetic valve 10 can include an outer skirt mounted on the outside of the frame in lieu of or in addition to the inner skirt 34 to further seal the prosthetic valve against surrounding tissue. The inner and/or outer skirts can be made of any of various suitable materials, including natural tissue (e.g., pericardium tissue) or any of various synthetic materials, which may be woven, non-woven, braided, knitted, and/or combinations thereof. In one specific implementation, the inner skirt 34 is made of a polyethylene terephthalate (PET) fabric.

Exemplary configurations of the prosthetic heart valve are further disclosed in U.S. Patent Application Publication Nos. 2014/0343670, 2012/0123529, 2010/0036484, and 2010/0049313.

Prosthetic heart valve 10, or another type of implantable, expandable medical device, such as an expandable stent, can be delivered to an implantation site via a delivery apparatus. An exemplary embodiment of such a delivery apparatus 100 is shown at FIGS. 2-9B.

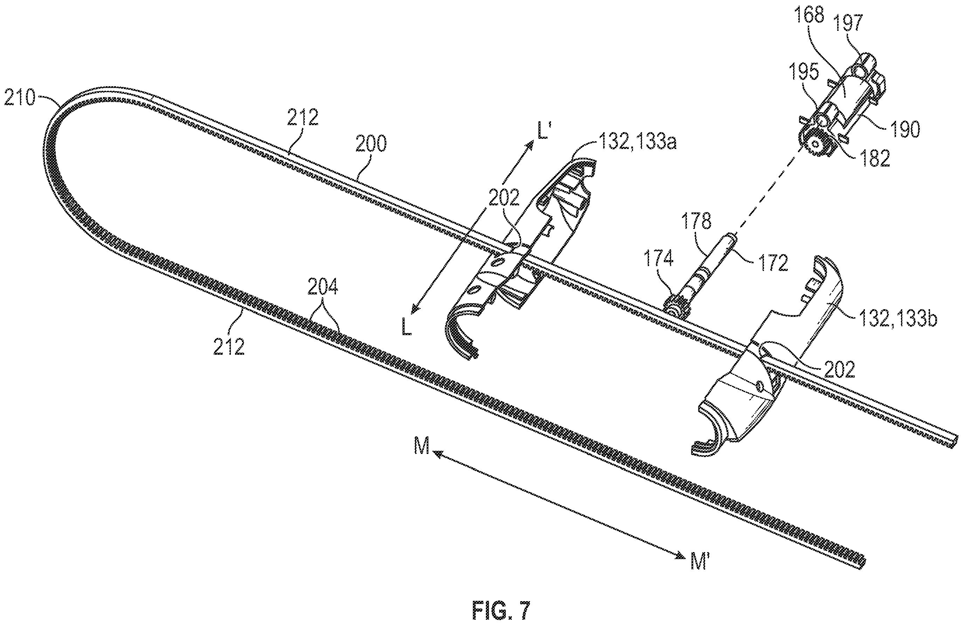

As shown in FIGS. 2 and 3, the delivery apparatus 100 comprises a handle portion 132 and can comprise a first shaft 134 extending distally therefrom. A user, such as a physician or clinician, can operate the delivery apparatus 100 via actuation of a plurality of knobs 136, dials, and/or buttons 138 located on the handle portion 132. The first shaft 134 has a proximal end portion 140 and a distal end portion 142. The proximal end portion 140 of the first shaft 134 can be coupled to the handle portion 132. The handle portion 132 can comprise a housing 133 that can include two housing portions 133a, 133b (as best shown in FIG. 7).

As shown in FIG. 3, the delivery apparatus 100 can include a second shaft 150 and a third shaft 152. The second shaft 150 extends distally from the handle portion 132 and co-axially through the first shaft 134. The third shaft 152 extends distally from the handle portion 132 and co-axially though the second shaft 150. In the illustrated, the first shaft 134 is the outermost shaft of the delivery apparatus and therefore can be referred to as the outer shaft 134 of the delivery apparatus. In the illustrated embodiment, the third shaft 152 is the innermost shaft of the delivery apparatus and therefore can be referred to as the inner shaft 152 of the delivery apparatus. In the illustrated embodiment, the second shaft 150 is intermediate or between the innermost shaft and the outermost shaft and therefore can be referred to as an intermediate shaft.

A nosecone 144 can be connected to or mounted on a distal end portion 152d of the inner shaft 152. The nosecone 144 can have a tapered outer surface as shown for atraumatic tracking of the delivery apparatus 12 through a subject's vasculature. The inner shaft 152 extends distally beyond the intermediate shaft 150, through a lumen of a cord manifold 120 and through the prosthetic valve 10.

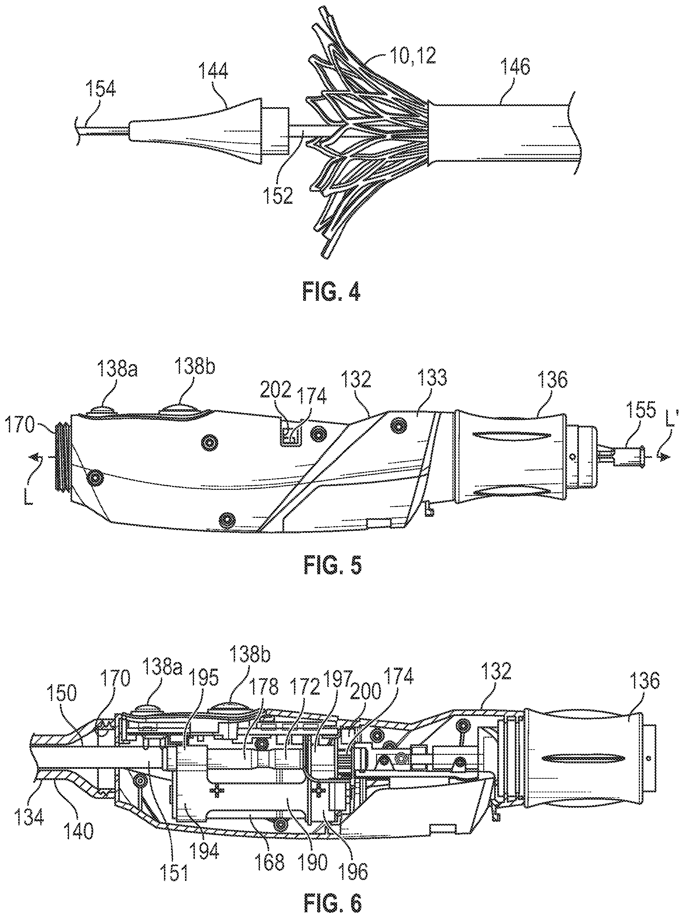

In certain embodiments, the first, second, and third shafts 134, 150, and 152, respectively, can be configured to be moveable relative to each other, including relative axial movement (in the proximal and distal directions) and/or relative rotational movement (in the clockwise and counterclockwise directions). A guide wire 154 (FIG. 4) can extend through the central lumen of the inner shaft 152 and the inner lumen of the nosecone 144 so that the delivery apparatus 100 can be advanced over the guide wire 154 inside the subject's vasculature during delivery of the prosthetic valve 10. The guide wire 154 may be inserted into the inner shaft 152 via a proximal port 155 of the handle portion 132 (FIG. 5).

A delivery capsule 146 is coupled to the distal end portion 142 of the first shaft 134, proximal to the nosecone 144. The delivery capsule 146 houses the prosthetic valve 10 therein in a radially compressed state, as shown at FIGS. 3-4. In one embodiment, the delivery capsule 146 covers and retains the underlying compressed prosthetic valve of FIG. 1. The delivery apparatus 100 is particularly suited for delivering and implanting a self-expandable prosthetic valve 10 that radially expands to its functional size under its own resiliency when deployed from the delivery capsule 146.

However, the prosthetic heart valve 10 alternatively can be a plastically expandable prosthetic valve or a mechanically expandable heart valve. If the delivery apparatus is used to implant a plastically expandable valve, the delivery apparatus can include a balloon catheter as known in the art for expanding the prosthetic valve, such as disclosed in U.S. Publication No. 2009/0281619. If the delivery apparatus is used to implant a mechanically expandable valve, the delivery apparatus can include one or more actuators for expanding the prosthetic valve, such as disclosed in U.S. Application No. 62/945,039, filed December 6, 2019.

As shown at FIG. 3, the delivery capsule 146 is configured to accommodate the prosthetic heart valve 10, or another type of implantable medical device, in a radially compressed state for delivery into a subject. The cord manifold 120 is configured to form a releasable connection with the prosthetic heart valve 10 via a plurality of cords or tethers 118. The cord manifold 120 is coupled to a distal end of the first shaft 134 proximal to each of the nosecone 144 and the crimped prosthetic valve 10.

The cord manifold 120 can include a proximal portion 122 and a distal portion 124 spaced axially from the proximal portion 122. The proximal portion 122 of the cord manifold 120 can be fixedly secured to the distal end portion 142 of the first shaft 134 using suitable techniques or mechanisms, such as via mechanical connectors, welding, a press-fit, and/or an adhesive. For example, in some embodiments, the distal end portion 142 of the shaft 134 can extend into a lumen of the proximal portion, which can be secured to the shaft 134 using any of the connection techniques described above.

The cords 118 may be made of any of various suitable biocompatible materials for use within a subject. In certain embodiments, a cord 118 can comprise a single filament cord or a multifilament or multi-strand cord formed from braiding, weaving, knitting, twisting, and wrapping a plurality of filaments or strands together. The filaments or strands can comprise polymeric fibers, such as ultra-high molecular weight polyethylene, nylon, polyester, and/or aramid, or flexible wires (e.g., metal wires).

Each of the cords 118 can have a first end 118a attached to the cord manifold 120, such as to the proximal portion 122. Each cord 118 extends through an opening of the frame 12 of the prosthetic valve (e.g., through an opening 26) and can have a second end 118b in the form of a loop that is retained on a release member 156. The release members 156 are configured to retain the cords 118 in a state connected to the frame 12 of the prosthetic valve 10 until they are actuated by a user to release the cords 118. Two release members 156 are shown for purposes of illustration. It should be understood that any number of release members 156 can be used.

Similarly, two cords 118 are shown for purposes of illustration, but it should be understood that any number of cords can be used. Also, there need not be an equal number of cords and release members 156. For example, the ends 118b of multiple cords 118 may be retained on a single release member. Desirably, at least three cords 118 are used to balance the attachment of the frame 12 to the cord manifold 120. In particular embodiments, the number of cords 118 is equal to the number of apices 18 of the frame 12 of the prosthetic valve 10 (FIG. 1). Moreover, in other embodiments, a single cord can be used to connect the frame 12 to the cord manifold 120 at multiple locations along the outflow end of the frame by forming multiple passes extending through openings of the frame.

Each release member 156 can extend in a slideable manner through respective openings in the proximal portion 122 and the distal portion 124 of the cord manifold 120. Each release member 156 can extend through the first shaft 134 along its entire length and can have proximal end portions that are operatively coupled to a knob 136 on the handle to control movement of the release members. Each of the release members 156 is moveable in the proximal and distal directions relative to the proximal and distal portions 122, 124, of the cord manifold between a distal position where each release member 156 retains a respective cord 118 and a proximal position where each release member 156 is released from a respective cord 118.

Further details regarding the attachment of the prosthetic valve 10 to the delivery apparatus 100 via one or more cords or sutures are disclosed in U.S. Publication Nos. 2014/0343670, 2012/0239142, and 2010/0049313 and U.S. Application No. 62/824,710, filed March 27, 2019.

Moreover, in alternative embodiments, different valve-retaining mechanisms can be used to form a releasable connection between the prosthetic valve 10 and the delivery apparatus 100. For example, in some embodiments, the posts 24 of the frame 12 can be retained in corresponding recesses of a shaft or retaining member of the delivery apparatus, which allow the posts of the frame to expand out of their corresponding recesses when the capsule 146 is retracted to deploy the prosthetic valve. In other embodiments, the retaining mechanism can comprise inner and outer metal fork members that form a release connection between the delivery apparatus and the prosthetic valve. Further details regarding alternative valve-retaining mechanisms are disclosed in 2012/0239142 and 2010/0049313.

As further shown in FIG. 3, the second shaft 150 can include an externally threaded portion 162 along a distal end portion thereof. The threaded portion 162 can comprise threads formed on the external surface of the shaft or can be a separate screw connected to the distal end of a proximal shaft section. The capsule 146 is operatively connected to the second shaft 150 by an internally threaded nut 164 disposed on the threaded portion 162. The nut 164 can have a radially extending projection 166 that extends into a corresponding opening in the capsule 146 (see FIG. 2). Rotation of the nut 164 is restricted by one or more rails 165 extending from or formed along the distal end portion of the first shaft 134.

Rotation of the second shaft 150 relative to the first shaft 134 therefore produces axial movement of the nut 164 (in the distal and proximal directions), which in turn produces corresponding axial movement of the capsule 146 in the same direction during loading, deployment, and/or recapture of the prosthetic valve. For example, when the nut 164 is in a distal position, the delivery capsule 146 extends over and retains the prosthetic valve 10 in a compressed state for delivery. Movement of the nut 164 in a proximal direction causes the delivery capsule 146 to move in the proximal direction, thereby deploying the prosthetic valve. Rotation of the second shaft 150 can be achieved via a motor operatively coupled to the second shaft and/or manual control features, as further described below.

In certain embodiments, the delivery apparatus 12 may comprise one or more steering mechanisms configured to control the curvature of one or more of the shafts 134, 150, 152 to assist in steering the delivery apparatus through a vasculature. For example, the steering mechanism can comprise one or more eccentrically positioned pull wires extending through a shaft and operatively connected to an adjustment mechanism located on or adjacent the handle portion 132. Adjustment of the adjustment mechanism is effective to change the tension of the pull wires to cause the shaft to curve in a given direction, or to straighten. In one implementation, one or more pull wires extend though the outer shaft 134 and adjustment of the adjustment mechanism is effective to adjust the curvature of the distal end portion of the outer shaft 134 and the delivery apparatus 100. Further details regarding the steering mechanism are disclosed in U.S. Publication Nos. 2007/0005131 and 2013/0030519.

In certain embodiments, as shown in FIGS. 6-11, the delivery apparatus 100 is a motorized apparatus comprising a motor 168 housed inside the handle portion 132. The motorized embodiment automates deployment of the prosthetic valve 10. In particular, the motor 168 is operatively coupled to the second shaft 150 to produce rotation of the second shaft 150 relative to the first shaft 134 and corresponding axial movement of the capsule 146, as further described below.

The proximal end portion 140 of the first shaft 134 can be coupled to a distal end of the handle portion 132. As shown at FIG. 6, a proximal end portion 151 of the second shaft 150 can extend into the handle portion 132 via a distal opening 170 of the handle portion 132. A rotatable component 172 (which can be referred to as a drive cylinder in some embodiments) is disposed inside the handle portion 132 and operatively coupled to the second shaft 150.

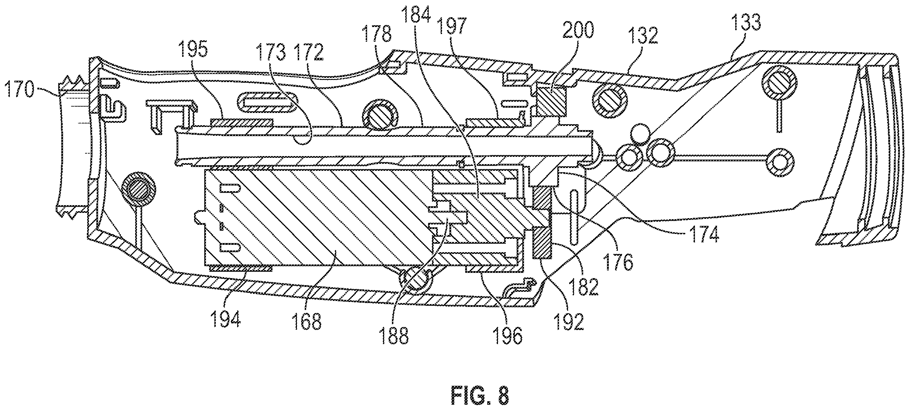

In one embodiment, as best shown at FIGS. 7-9, the proximal end portion of the rotatable component comprises a gear 174 having a plurality of gear teeth 176 that are circumferentially arrayed relative to each other. The rotatable component 172 further comprises a main body 178 configured as an extended shaft having a lumen 173 (FIG. 8). In the illustrated embodiment the main body 178 and gear 174 are integrally formed, although they may be separately formed components that are connected to each with any of various attachment means. The main body 178 of the rotatable component 172 can be coaxial to a central longitudinal axis L-L' (shown in FIG. 5) of the handle portion 132 and also coaxial to the first shaft 134. The lumen 173 of the main body 178 can be sized to receive and hold the proximal end portion 151 of the second shaft 150 therein.

In some implementations, the inner surface of the lumen 173 can have a non-circular cross-section in a plane perpendicular to the longitudinal axis L-L' and the proximal end portion 151 of the second shaft 150 can have a similar cross-sectional profile that corresponds to the shape of the lumen so that rotational motion of the rotatable component 172 is transferred to the second shaft 150. For example, the lumen 173 and the proximal end portion 151 can be generally cylindrical and have a series of circumferentially spaced flat sections. In lieu of or in addition to providing the lumen 173 and proximal end portion 151 with non-circular cross-sections, the proximal end portion 151 can be secured to the rotatable component with fastening means, such as mechanical fasteners (e.g., a screw), adhesives, a press fit, a snap fit connection, etc.

As best shown in FIG. 6, the motor 168 may be held inside a retaining case or cradle 190. The motor can be an electric motor and the handle portion can include a battery compartment that contains one or more batteries (not shown) for powering the motor 168. One or more operator buttons 138a, 138b on the handle portion allow a user to activate the motor, such as by electrically coupling current from the battery power to the motor. The motor can be rotated in either direction, as described below, thereby moving the capsule 146 in either the proximal or distal direction. One of the buttons (e.g., button 138a) can be operable to rotate the motor in a first rotational direction to move the capsule 146 in a distal direction, such as for loading the prosthetic valve in the capsule, and the other button (e.g., button 138b) can be operable to rotate the motor in a second rotational direction to move the capsule in a proximal direction, such as for deploying the prosthetic valve. In lieu of or in addition to one or more batteries, the motor 168 can be configured to receive a power cord that supplies electric current to the motor from a power source external to the handle portion (e.g., a wall outlet).

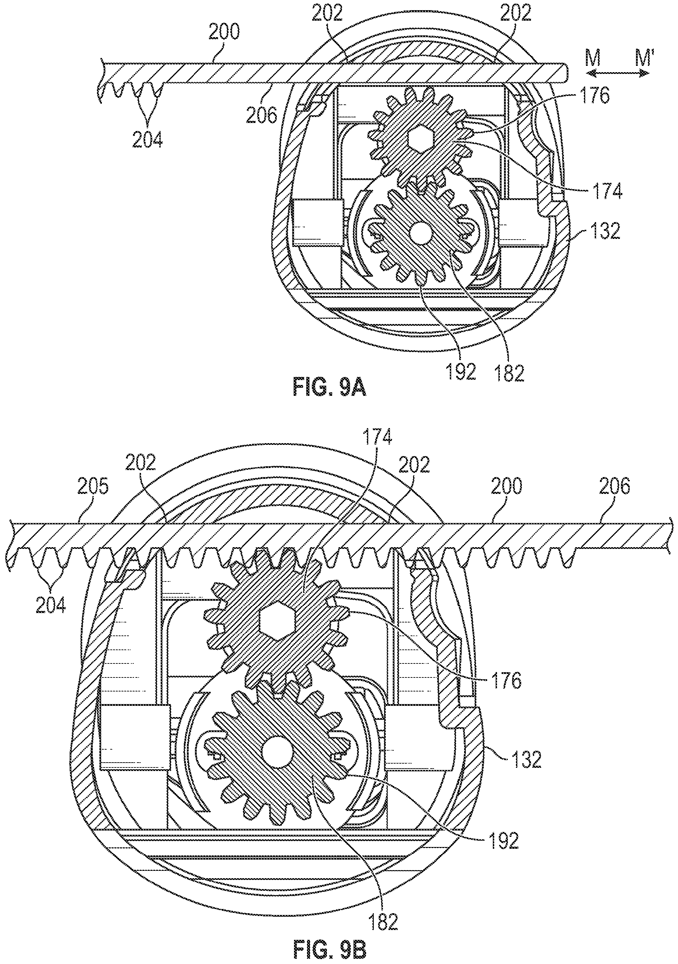

As best shown in FIG. 8, the motor 168 can be coupled to the rotatable component 172 by a drive shaft 184 connected to a motor shaft 188 and an intermediate drive gear 182 connected to the drive shaft 184. The drive gear 182 can have circumferentially arrayed gear teeth 192 that can engage with the circumferentially arranged gear teeth 176 of the gear 174 of the rotatable component 172, as shown in FIGS. 9A-9B. When driven by the motor, the motor 168 rotates the motor shaft 188, which in turn rotates the drive shaft 184 and the drive gear 182. The drive gear 182 engages and rotates the gear 174 of the rotatable component 172, thereby rotating the rotatable component 172 and the second shaft 150. The drive gear 182 can be positioned radially offset from a central axis of the rotatable component 172 and a central longitudinal axis L-L' of the handle portion 132 such that when meshed, the gears are vertically aligned, as shown in FIGS. 9A-9B. In further embodiments, one or more additional gears may be provided between the drive gear and the rotatable component to transfer rotation from the motor to the rotatable component.

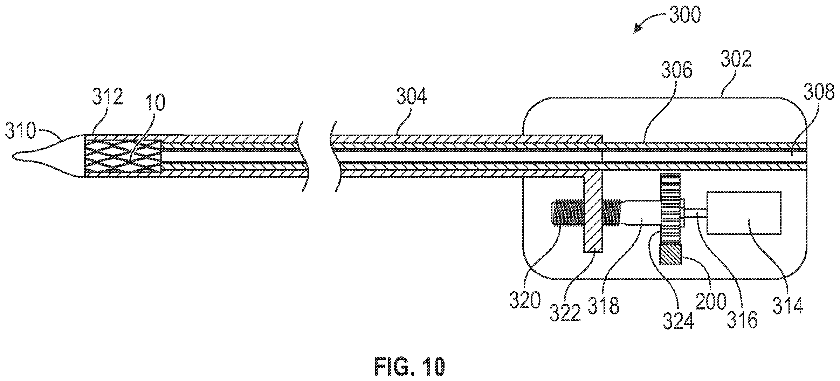

In alternative embodiments, the motor shaft 188 or the drive shaft 184 can be connected to the rotatable component 172 without any intervening gears. For example, the motor shaft 168 can be positioned proximal to the rotatable component along the axis L-L' and the motor shaft 188 can be connected to the rotatable component 172 in a direct drive arrangement (see, e.g., the embodiment of FIG. 10, described below).

FIGS. 9A-9B show a cross-sectional view of the handle portion 136 depicting the meshing of gear teeth 192 of drive gear 182 with gear teeth 176 of the rotatable component 172. In addition, the figure shows the vertical alignments of the gears relative to each other. In other embodiments, the positioning may be reversed. The vertical alignment of the gears allows for the components to be operably coupled while being housed in the limited space available in the handle portion.

In the illustrated embodiment, the cradle 190 housing the motor 168 and the drive shaft 184 may also be configured to support the rotatable component 172 for rotational movement within the handle portion. As best shown in FIGS. 6-7, the cradle 190 may have a first distal portion 194 that includes a distal sleeve 195 encircling a distal end portion of the main body 178 of the rotatable component 172. The cradle 190 may further have proximal portion 196 that includes a proximal sleeve 197 encircling a proximal end portion of the main body 178 of the rotatable component 172.

With reference to FIGS. 3, 8, 9A, and 9B, rotation of the motor 168 in a first direction (e.g., clockwise or counterclockwise) causes rotation of the rotatable component 172. This in turn causes rotation of the second shaft 150, which is coupled to the rotatable component 172. Rotation of the second shaft 150 causes rotation of the screw 162 of the second shaft 150. As described above, rotation of the screw 162 produces axial movement of the drive nut 164 and the capsule 146 (FIG. 3). For example, rotation of the rotatable component in the first direction can cause the delivery capsule 146 to retract in a proximal direction and uncover the prosthetic valve at the distal end of the delivery apparatus. Conversely, rotation of the motor in a second direction, opposite to the first direction, causes the second shaft 150 to rotate in an opposite direction, which causes the nut to move axially in the opposite direction, moving the delivery capsule in a distal direction back over the prosthetic valve. An operator can actuate buttons 138a, 138b (FIG. 6) on the handle portion to activate the motor, and axially move the delivery capsule in a motorized manner. This allows for quick deployment or retrieval of the prosthetic valve.

In use, the prosthetic valve 10 can be connected to the delivery apparatus 100 and loaded into the capsule as follows. A releasable connection can be formed between each apex 18 at one end of the frame 12 and the cord manifold 120 with a separate cord 118. Optionally, the length of the cords 118 are selected such that the secured end of the frame is held in an at least partially radially compressed state by the cords. After securing the end of the frame 12 with the cords 118, the delivery capsule 146 can be advanced distally (e.g., by pressing button 138a) over the cord manifold 120, the cords 118, and the frame 12, causing the frame to collapse to a radially compressed state under the force of the capsule 146 (as shown in FIG. 4). The delivery capsule 146 is advanced distally until the distal end of delivery capsule 146 abuts the nosecone 144 to fully enclose the prosthetic valve 10, as shown in FIG. 3.

After loading the prosthetic heart valve 10 within the delivery apparatus 12 as described above, the delivery apparatus can be inserted in the vasculature and advanced or navigated through the vasculature to the desired implantation site (e.g., through a femoral artery and the aorta when delivering the prosthetic valve 10 in a retrograde delivery approach to the native aortic valve).

Once the prosthetic valve 10 is delivered to a selected implantation site within the subject (e.g., the native aortic valve), the nose cone 144 optionally can be advanced distally away from the adjacent end of the capsule 146 by pushing the inner shaft 152 distally to avoid contact between the prosthetic valve and the nose cone during valve deployment. The delivery capsule 146 may be retracted (e.g., by pressing button 138b) in order to deploy the prosthetic valve 10. As the delivery capsule 146 is retracted (FIG. 4), the prosthetic valve can radially self-expand under the resiliency of the frame 12. After the delivery capsule 146 is fully retracted from the prosthetic valve 10, the prosthetic valve is still attached to the delivery apparatus 12 by the cords 118. While still attached to the delivery apparatus, the user can manipulate the delivery apparatus (e.g., by moving it in the proximal and distal directions and/or rotating it) to adjust the position of the prosthetic valve relative to the desired implantation location.

If desired, the delivery capsule can be advanced back over the prosthetic valve 10 to fully or partially recapture the prosthetic valve (bring the prosthetic valve back within the capsule) to facilitate re-positioning of the prosthetic valve. For example, after crossing the native aortic valve leaflets in a retrograde delivery approach and deploying the prosthetic valve, it may be desirable to recapture the prosthetic valve back within the capsule, retract the delivery apparatus to bring the prosthetic valve back within the aorta, and then advance the prosthetic valve back across the native aortic valve leaflets, and deploy the prosthetic valve from the capsule.

Once the prosthetic valve is deployed from the capsule 146 and positioned at the desired implantation location, the release members 156 can be retracted, such as by rotating the knob 136 on the handle portion 132. In some cases, the cords 118 slide outwardly from the apertures 26 and free themselves from the frame 12 by virtue of the self-expanding frame 12 further expanding when the release members 156 are retracted. In other cases, the user can slightly retract the delivery apparatus 100, which in turn pulls the cords 118 proximally relative to the frame 12 to pull them out of the apertures 26.

Optionally, the orientation of the prosthetic valve can be reversed such that the inflow end of the prosthetic valve is the proximal end and the outflow end of the prosthetic valve is the distal end when coupled to the delivery apparatus. This can facilitate delivery of the prosthetic valve to different implantation locations (e.g., the native aortic, pulmonary, mitral, and tricuspid annuluses) and/or for various delivery approaches (e.g. antegrade, transseptal, trans ventricular, transatrial).

As discussed above, rotatory movement of the rotatable component 172 produces axial movement of the second shaft 150 and the delivery capsule 146. While rotation of the rotatable component 172 can be affected by the motor 168, the rotation can also be achieved manually.

To such ends, the delivery apparatus 100 is provided with a manual deployment tool that enables the rotatable component to be manually operated, and the prosthetic valve to be deployed or recaptured, in the event of motor or battery malfunction. For example, if the motor or battery fails while a user is in the midst of operating the motor to rotate the rotatable component, the user has only a limited amount of time in which to complete retraction of the delivery capsule or to recapture the prosthetic valve. An exemplary embodiment of such a manual deployment tool 200 is shown in FIGS. 7, 9A, and 9B.

As best shown in FIGS. 5-9, the handle portion 132 may include one or more openings 202 in the housing 133 through which the manual deployment tool 200 can be inserted to manually rotate the rotatable component 172. In the depicted embodiment, the manual deployment tool 200 is in the form of a pull cord (which can also be referred to as a pull belt or toothed member). To operate the rotatable component 172 manually, the pull cord 200 is inserted into the opening 202 and pulled through the handle portion 136 along an axis M-M', which can be offset from the longitudinal axis L-L' of the handle portion (FIGS. 7 and 9A). As shown in FIGS. 7 and 9A-9B, the pull cord 200 can be inserted through the opening 202 in a direction that is substantially perpendicular to the lengthwise axis (e.g., longitudinal axis L-L') of the handle portion 132. In the depicted embodiments, the manual deployment tool 200 is a pull cord that is separable (e.g., insertable and removable) from the handle portion 132 of the delivery apparatus.

In some embodiments, the handle portion 132 can include a removable cover or plug that extends over and covers the opening 202 in the housing 133. The cover can remain in place during normal use until the pull cord 200 is needed, at which time the cover can be removed to provide access to the opening 202.

As shown in FIGS. 9A-9B, at least a portion 205 of the pull cord 200 comprises a plurality of teeth 204 that are configured to drivingly engage the plurality of gear teeth 176 of the rotatable component 172 as the pull cord 200 is pulled through the opening 202 in the handle portion 132. In this manner, the pull cord 200 functions as a linear rack that produces rotation of the rotatable component 172 upon linear movement of the pull cord along axis M-M'. As shown in FIG. 7, the pull cord 200 can have teeth 204 along a majority of the length of the pull cord 200.

The pull cord 200 can have a length in a range of about 12 inches to about 36 inches, a range of about 16 inches to about 30 inches, or a range of about 43 cm to 51 cm ( about 17 inches to about 20 inches). In some embodiments, a total length of the pull cord 200 can be 46 cm (18 inches).

The pull cord 200 can be made from any of various suitable materials, including metals, polymers, etc. In some embodiments, the pull cord 200 can be made from molded plastic and can have a curved portion 210 connecting two straight portions 212, as shown in FIG. 7. The pull cord 200 can be sufficiently flexible such that the curved portion 210 can straighten as the pull cord 200 is being pulled though the opening 202 in the handle portion 132. In some embodiments, the pull cord 200 can be straight along its length from one end to the other end of the pull cord. In other embodiments, the pull cord 200 can be molded into a coil or spiral shape that can unwind or straighten as it is being pulled through the opening 202 in the handle portion 132. Moreover, in some embodiments, the pull cord 200 can be sufficiently rigid such that it can be pushed through the handle portion 132 during use in lieu of (alternative "in lieu of" is not covered by the presently claimed invention) or in addition to being pulled through the handle portion. Whether the pull cord is pushed or pulled through the handle portion, manually moving the pull cord through the handle portion operates the rotatable component 172, as further described below.

In some embodiments (see FIG. 9A), at least one end portion 206 of the pull cord may be without any teeth 204 and can have a flat surface facing the gear teeth 176 as shown. The end portion 206 desirably can be sized such that it can be inserted through the opening 202 without contacting the gear teeth 176 or at least easily inserted through with minimal resistance from the gear teeth 176. This allows the end portion 206 to be more easily inserted through the opening 202 from one side of the handle portion (the left side in FIGS. 9A-9B) to the other side of the handle portion (the right side in FIGS. 9A-9B) before any of the teeth 204 of the pull cord 200 engage the gear teeth 176. As the end portion 206 emerges from the other side of the handle portion 132 (the right side in FIGS. 9A-9B), the end portion 206 can then be grasped by the user to pull the pull cord 200 further through the opening 202 (to the right in FIGS. 9A-9B) until the teeth 204 engage the gear teeth 176.

Movement of the pull cord 200 through the opening 202 in a first direction (e.g., a first direction along M-M') rotates the rotatable component 172 in a first rotational direction (e.g., clockwise or counterclockwise) while movement of the pull cord through the opening in a second rotational direction, opposite the first direction (e.g., opposite direction along M-M') rotates the rotatable component 172 in a second direction, opposite the first direction. Since the rotation of the rotatable component results in the axial movement of the delivery capsule, movement of the pull cord in the first direction along axis M-M' can be used to retract the delivery capsule 146 and deploy the prosthetic valve 10 while movement of the pull cord the second direction along the axis M-M' can be used to advance the delivery capsule 146 distally and recapture the prosthetic valve 10.

During a prosthetic valve delivery procedure, a user can insert the prosthetic valve 10 in the subject with the delivery apparatus 100, with the prosthetic valve 10 retained in a radially compressed state within the delivery capsule 146 as previously described. The user can then operate an electric switch (e.g., button 138b) to actuate the motor 168 housed in the handle portion 132 of the delivery apparatus. If the motor fails to rotate and produce axial movement of the delivery capsule 146, the user can then insert and pull the pull cord 200 through the opening 202 of the handle portion 132 (e.g., from left to right in FIGS. 9A-9B) to produce rotation of the rotatable component 172 inside the handle portion 132. This in turn rotates the second shaft 150 and produces axial movement of the delivery capsule 146 in the proximal direction relative to the prosthetic valve to deploy the prosthetic valve from the delivery capsule and allow the prosthetic valve to radially expand from the radially compressed state to a radially expanded state. In some embodiments, once the prosthetic valve is radially expanded and deployed from the delivery apparatus 100, the pull cord 200 can be removed from the handle portion 132.

If, during an implantation procedure, the motor fails to completely retract and deploy the prosthetic valve and it is desired to recapture the prosthetic valve back within the delivery capsule, the pull cord 200 can be inserted into the opening 202 in the opposite direction (e.g., from right to left in FIGS. 9A-9B) and pulled through the opening to rotate the rotatable component in the opposite direction and produce axial movement of the delivery capsule 146 in the distal direction to move the delivery capsule back over the prosthetic valve. Once the prosthetic valve is within the delivery capsule, the delivery apparatus 100 can be used to reposition the prosthetic valve for deployment or it can be withdrawn from the body completely.

In this way, a manual deployment tool is provided for manually operating a rotatable component of a delivery apparatus that enables axial movement of a delivery capsule. Consequently, if a motor driving the rotatable component fails during a valve deployment procedure, a user can drive the rotatable component by operating the manual deployment tool and complete the procedure. In this way, the manual deployment tool acts as a simple, back-up or bail out tool that ensure a higher completion efficiency of implantation procedures.

In an alternative embodiment, a pull cord 200 can extend through housing 133 of the handle portion 132 at a location to engage the drive gear 182 instead of drive gear 174. In such an embodiment, the pull cord 200 is used in the same manner as described above except that the pull cord 200 is pulled through the housing to rotate the gear 182, which in turn rotates the gear 174 and the rotatable component 172.

FIG. 10 shows a motorized delivery apparatus 300 that can be operated by way of a pull cord 200, according to another embodiment. The delivery apparatus 300 in the illustrated embodiment comprises a handle portion 302, a first, outer shaft 304 extending distally from the handle portion 302, a second, intermediate shaft 306 extending distally from the handle portion 302 through the first shaft 304, and a third, inner shaft 308 extending distally from the handle portion 302 through the second shaft 306. A nose cone 310 can be mounted on a distal end portion of the inner shaft 308.

In the embodiment of FIG. 10, a distal end portion 312 of the first shaft 304 serves as a delivery capsule. A prosthetic valve 10 can be releasably connected to a distal end portion of the second shaft 306 using any of the retaining mechanisms described above. The prosthetic valve 10 can be deployed from the delivery capsule 312 by retracting the first shaft 304 relative to the prosthetic valve and the second shaft 306.

The handle portion 302 can house a motor 314 having a shaft 316 that is connected to a lead screw 318. The lead screw 318 can have an externally threaded portion 320 that extends through and threadably engages a threaded opening of an extension portion 322 of the first shaft 304. The extension portion 322 serves as a nut that can travel in the proximal and distal directions upon rotation of the lead screw. The handle portion 302 can have other features described in connection with the delivery apparatus 100 for operating the motor 314. For example, the handle portion 302 can house one or more batteries and can have one or more buttons or switches (e.g., buttons 138a, 138b) for actuating the motor 314.

During an implantation procedure, a user can advance the nose cone 310 distally away from the delivery capsule 312 by pushing the inner shaft 308 distally once the prosthetic valve is positioned at the implantation site. The user can actuate the motor 314 (such as by pressing button 138b), which rotates the lead screw 318 and retracts the first shaft 304 in the proximal direction (to the right in FIG. 10) and uncovers the prosthetic valve. Operating the motor 314 in the reverse direction (such as by pressing button 138a) rotates the lead screw in the opposite direction and moves the first shaft 304 back over the prosthetic valve if recapture and/or repositioning is required.

To manually operate the lead screw 318, a gear 324 having a plurality of gear teeth can be mounted on the lead screw 318. The handle portion 302 can include an opening to receive the pull cord 200 at a location where the teeth 204 of the pull cord 200 can engage the teeth of the gear 324. Pulling the pull cord 200 through the handle portion 302 in a first direction is effective to rotate the gear 324 and the lead screw 318 in a first rotational direction to retract the first shaft 304. Pulling the pull cord 200 through the handle portion 302 in a second direction, opposite the first direction, is effective to rotate the lead screw 318 in a second rotational direction to move the first shaft 304 in a distal direction.

In alternative embodiments, in lieu of a gear 324, gear teeth can be provided directly on an external surface of the lead screw for engaging the teeth 204 of the pull cord.

Further, it should be noted that any moveable component of a delivery apparatus 100, 300 can be configured to be operated by a pull cord 200. In one implementation, for example, the lead screw 318 can engage an extension portion of the second shaft 306, in which case the prosthetic valve 10 can be deployed by moving the second shaft 306 and the prosthetic valve 10 distally relative to the first shaft 304. The motor 314 and the pull cord 200 can be used to produce movement of the second shaft 306 in the distal and proximal directions, depending on whether the prosthetic valve is to be deployed or recaptured back into the capsule 312.

In alternative embodiments, the pull cord 200 can be have other configurations. For example, in one implementation, the pull cord 200 can have teeth 204 along both sides of the pull cord. In another implementation, instead of teeth 204, the pull cord 200 can have helically extending external threads (similar to a screw) along the length of the pull cord. The gear (e.g., gear 174) or other delivery apparatus component designed to engage the pull cord can have corresponding features that mate with the helical threads of the pull cord and produce rotation of delivery apparatus component when the pull cord is pulled relative to the delivery apparatus component.

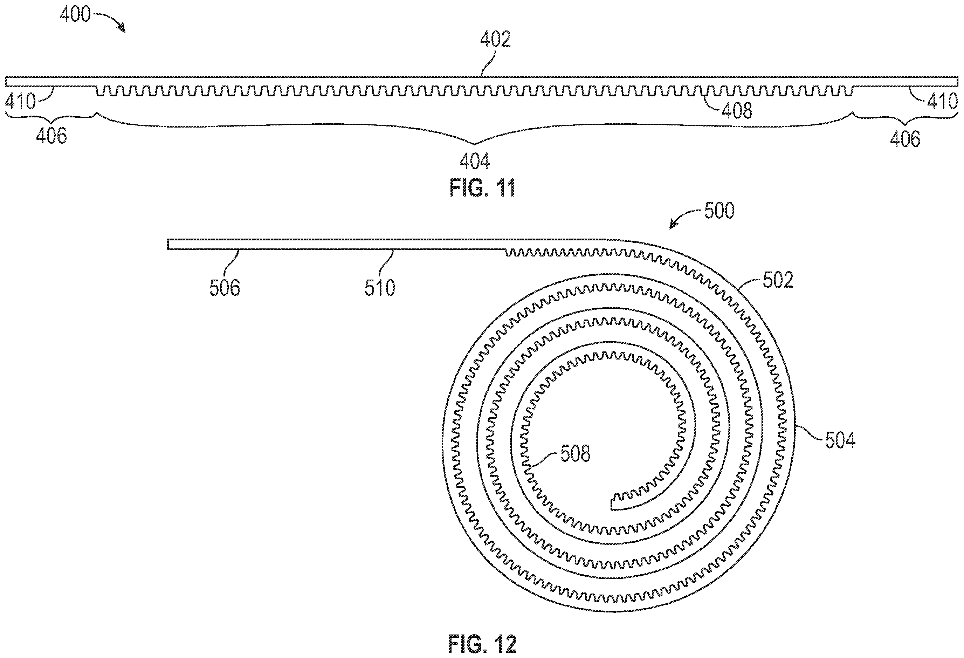

Another exemplary embodiment of a manual deployment tool, configured as a pull cord 400, is shown in FIG. 11. The pull cord 400 can be configured and function similar to the pull cord 200 (as described above with reference to FIGS. 7, 9A, and 9B), to manually deploy a prosthetic valve from a delivery apparatus (such as the delivery apparatus 100 or 300).

As shown in FIG. 11, the pull cord 400 is straight (e.g., without curves, bends, or coils) along its length from one end to the other end of the pull cord 400. For example, the pull cord 400 has a straight body 402 comprising a middle portion 404 disposed between two end portions 406. The middle portion 404 comprises a plurality of teeth 408 (e.g., similar to teeth 204 of the pull cord 200 shown in FIGS. 7, 9A, and 9B). The end portions 406 do not include teeth and have a relatively smooth (e.g., untoothed) surface 410.

In alternate embodiments, the pull cord 400 can include only one untoothed end portion 406 and a remainder of the pull cord 400 can include teeth 408.

The pull cord 400 can have a length in a range of about 30 cm to 91 cm (about 12 inches to about 36 inches), a range of about 41 cm to 76 cm about 16 inches to about 30 inches), or a range of about 43 cm to 51 cm (about 17 inches to about 20 inches). In some embodiments, a total length of the pull cord 400 can be 46 cm (18 inches).

FIG. 12 shows another exemplary embodiment of a manual deployment tool, configured as a pull cord 500. The pull cord 500 can be configured and function similar to the pull cord 200 (as described above with reference to FIGS. 7, 9A, and 9B), to manually deploy a prosthetic valve from a delivery apparatus (such as the delivery apparatus 100 or 300).

As shown in FIG. 12, the pull cord 500 is shaped as a coil or spiral. Said another way, the pull cord 500 can be coiled along at least a portion of its length. For example, the pull cord 500 can be molded into a coil or spiral shape which can unwind or straighten as it is being pulled through an opening in a handle portion of a delivery apparatus (e.g., the opening 202 in the handle portion 132 of delivery apparatus 100). The pull cord 500 comprises a body 502, the body 502 comprising a coiled, toothed portion 504 and an end portion 506. The toothed portion 504 is coiled and includes a plurality of teeth 508 along its length. The end portion 506 does not include teeth and has a relatively smooth (e.g., untoothed) surface 510. In some embodiments, as shown in FIG. 12, the end portion 506 is straight (e.g., not coiled).

The pull cord 500 can have a length in a range of about 30 cm to about 91 cm (about 12 inches to about 36 inches), a range of about 41 cm to about 76 cm (about 16 inches to about 30 inches), or a range of about 43 cm to about 51 cm (about 17 inches to about 20 inches).

In alternative embodiments, a pull cord, such as pull cord 200, 400, or 500, can be used to operate a delivery apparatus component that does not include a motor. For example, a delivery apparatus 100, 300 can be without a motor 168, 314 and instead the prosthetic valve 10 can be deployed from the delivery capsule 146, 312 solely by use of the pull cord.

Finally, it should be noted that the general concept of using a pull cord, such as the pull cord 200, 400, or 500, is used to produce movement of a moveable component of a medical apparatus having a handle and rotatable component inside the handle, with or without a motor inside the handle. The pull cord can be configured to cause rotation of the rotatable component, which in turn causes axial movement of the moveable component of the medical apparatus. The moveable component can be a delivery capsule as described above or another component of the medical apparatus, such as a shaft, a guidewire, or an instrument that is moved or deployed inside the body upon actuation of the pull cord.

In view of the many possible embodiments to which the principles of the disclosed technology may be applied, it should be recognized that the illustrated embodiments are only preferred examples of the disclosed technology and should not be taken as limiting the scope of the claimed subject matter. Rather, the scope of the claimed subject matter is defined by the following claims.

## Claims

1. A medical apparatus for insertion into a subject, the medical apparatus comprising:
a handle portion (132);
a moveable component (146) configured to be inserted into the subject;
a rotatable component (172) disposed in the handle portion (132) and operatively coupled to the moveable component (146) to produce axial movement of the moveable component (146) relative to the handle portion (132) upon rotation of the rotatable component (172);
a motor (168) disposed in the handle portion (132) and operatively coupled to the rotatable component (172) to produce rotation of the rotatable component (172) and corresponding axial movement of the moveable component (146); and
a pull cord (200) configured to produce rotation of the rotatable component (172) and corresponding axial movement of the moveable component (146) when a manual pulling force is applied to the pull cord (200) to pull the pull cord (200) relative to the rotatable component (172).

2. The medical apparatus of claim 1, wherein the moveable component (146) comprises a delivery capsule (146) configured to retain an implantable medical device (10) in a radially compressed state for delivery into the subject.

3. The medical apparatus of claim 1 or claim 2, wherein the handle portion (132) comprises an opening (202) for inserting the pull cord (200) through the handle portion (132) and wherein the pull cord (200) is configured to be pulled through the opening (202) in a direction substantially perpendicular to a lengthwise axis of the handle portion (132) to produce rotation of the rotatable component (172).

4. The medical apparatus of any preceding claim, wherein the rotatable component (172) comprises a plurality of gear teeth (176), wherein the pull cord (200) comprises a plurality of teeth (204) that are configured to drivingly engage the gear teeth (176) of the rotatable component (172), and wherein the pull cord (200) comprises a middle portion (404) comprising the plurality of teeth (408) and two end portions (406) without teeth, the middle portion (404) disposed between the two end portions (406).

5. A delivery apparatus (100) for an expandable, implantable medical device (10), the delivery apparatus (100) comprising the medical apparatus of claim 1 and further comprising a shaft (134) extending from the handle portion (132);
wherein the movable component (146) comprises a delivery capsule (146) configured to house the medical device (10) in a radially compressed state for delivery into a subject.

6. The delivery apparatus (100) of claim 5, wherein the shaft (134) is a first shaft (134) and the delivery apparatus (100) further comprises a second shaft (150) extending through the first shaft (134), wherein the second shaft (150) has a proximal end portion (151) operatively coupled to the rotatable component (172) and a distal end portion operatively coupled to the delivery capsule (146) such that rotation of the rotatable component (172) rotates the second shaft (150) relative to the first shaft (134) and produces axial movement of the delivery capsule (146).

7. The delivery apparatus (100) of claim 5, wherein the delivery capsule (146) is connected to a distal end portion (142) of the shaft (134) and the rotatable component (172) is operatively coupled to a proximal end portion (140) of the shaft (134) such that rotation of the rotatable component (172) produces axial movement of the shaft (134) and the delivery capsule (146).

8. The delivery apparatus (100) of any one of claims 5 to 7, wherein the handle portion (132) comprises an opening (202) for inserting the pull cord (200) through the handle portion (132),
optionally wherein the pull cord (200) is configured to be pulled through the opening (202) in a direction substantially perpendicular to a lengthwise axis of the handle portion (132) to produce rotation of the rotatable component (172).

9. The delivery apparatus (100) of claims 8, wherein movement of the pull cord (200) through the opening (202) in a first direction rotates the rotatable component (172) in a second direction to produce movement of the delivery capsule (146) in a third, proximal direction, and movement of the pull cord (200) through the opening (202) in a fourth direction, opposite the first direction, rotates the rotatable component (172) in a fifth direction, opposite the second direction to produce movement of the delivery capsule (146) in a sixth, distal direction.

10. The delivery apparatus (100) of any one of claims 5 to 9, wherein the motor (168) is an electrically actuated motor powered by at least one battery, which is housed in the handle portion (132).

11. The delivery apparatus (100) of any one of claims 5 to 10, wherein the pull cord (200) is removable from the handle portion (132).

12. The delivery apparatus (100) of any one of claims 5 to 11, wherein the rotatable component (172) comprises a plurality of gear teeth (176), and wherein the pull cord (200) comprises a plurality of teeth (204) that are configured to drivingly engage the gear teeth (176) of the rotatable component (172),
optionally further comprising a drive gear (182) coupled to the motor (168) and comprising a plurality of teeth (192) that engage the gear teeth (176) of the rotatable component (172).

13. The delivery apparatus (100) of claim 12, wherein the pull cord (200) comprises at least one end portion (406) without teeth.

14. The delivery apparatus (100) of any one of claims 5 to 13, wherein the pull cord (200) comprises a middle portion (404) comprising a plurality of teeth (408) and two end portions (406) without teeth, the middle portion (404) disposed between the two end portions (406).

15. The delivery apparatus (100) of any one of claims 5 to 14, wherein the pull cord (200):
a) is straight along its length; or
b) is coiled along at least a portion of its length and wherein a coiled portion (504) of the pull cord (200) comprises a plurality of teeth (508).

## Patentansprüche

1. Medizinische Vorrichtung zum Einführen in einen Patienten, wobei die medizinische Vorrichtung Folgendes umfasst:
einen Griffabschnitt (132),
eine bewegliche Komponente (146), die zum Einführen in einen Patienten ausgestaltet ist,
eine drehbare Komponente (172), die in dem Griffabschnitt (132) angeordnet und mit der beweglichen Komponente (146) wirkgekoppelt ist, um bei Drehung der drehbaren Komponente (172) eine axiale Bewegung der beweglichen Komponente (146) bezüglich des Griffabschnitts (132) zu erzeugen,
einen in dem Griffabschnitt (132) angeordneten und mit der drehbaren Komponente (172) wirkgekoppelten Motor (168), um eine Drehung der drehbaren Komponente (172) und eine entsprechende axiale Bewegung der beweglichen Komponente (146) zu erzeugen, und
eine Zugschnur (200), die dazu ausgestaltet ist, eine Drehung der drehbaren Komponente (172) und eine entsprechende axiale Bewegung der beweglichen Komponente (146) zu erzeugen, wenn die Zugschnur (200) mit einer manuellen Zugkraft beaufschlagt wird, um an der Zugschnur (200) bezüglich der drehbaren Komponente (172) zu ziehen.

2. Medizinische Vorrichtung nach Anspruch 1, wobei die bewegliche Komponente (146) eine Zuführkapsel (146) umfasst, die dazu ausgestaltet ist, eine implantierbare medizinische Vorrichtung (10) in einem radial komprimierten Zustand zur Zuführung in den Patienten zu halten.

3. Medizinische Vorrichtung nach Anspruch 1 oder Anspruch 2, wobei der Griffabschnitt (132) eine Öffnung (202) zum Einführen der Zugschnur (200) durch den Griffabschnitt (132) umfasst und wobei die Zugschnur (200) dazu ausgestaltet ist, in einer im Wesentlichen senkrecht zu einer Längsachse des Griffabschnitts (132) verlaufenden Richtung durch die Öffnung (202) gezogen zu werden, um eine Drehung der drehbaren Komponente (172) zu erzeugen.

4. Medizinische Vorrichtung nach einem der vorhergehenden Ansprüche, wobei die drehbare Komponente (172) eine Vielzahl von Zahnradzähnen (176) umfasst, wobei die Zugschnur (200) eine Vielzahl von Zähnen (204) umfasst, die dazu ausgestaltet sind, die Zahnradzähne (176) der drehbaren Komponente (172) in Antriebseingriff zu nehmen, und wobei die Zugschnur (200) einen mittleren Abschnitt (404) umfasst, der die Vielzahl von Zähnen (408) und zwei Endabschnitte (406) ohne Zähne umfasst, wobei der mittlere Abschnitt (404) zwischen den beiden Endabschnitten (406) angeordnet ist.

5. Zuführvorrichtung (100) für eine expandierbare, implantierbare medizinische Vorrichtung (10), wobei die Zuführvorrichtung (100) die medizinische Vorrichtung nach Anspruch 1 umfasst und ferner einen sich von dem Griffabschnitt (132) erstreckenden Schaft (134) umfasst,
wobei die bewegliche Komponente (146) eine Zuführkapsel (146) umfasst, die zur Aufnahme der medizinischen Vorrichtung (10) in einem radial komprimierten Zustand zur Zuführung in einen Patienten ausgestaltet ist.

6. Zuführvorrichtung (100) nach Anspruch 5, wobei der Schaft (134) ein erster Schaft (134) ist und die Zuführvorrichtung (100) ferner einen zweiten Schaft (150) umfasst, der sich durch den ersten Schaft (134) erstreckt, wobei der zweite Schaft (150) einen proximalen Endabschnitt (151), der an die drehbare Komponente (172) wirkgekoppelt ist, und einen distalen Endabschnitt hat, der an die Zuführkapsel (146) wirkgekoppelt ist, so dass durch die Drehung der drehbaren Komponente (172) der zweite Schaft (150) bezüglich des ersten Schafts (134) gedreht wird und eine axiale Bewegung der Zuführkapsel (146) erzeugt wird.

7. Zuführvorrichtung (100) nach Anspruch 5, wobei die Zuführkapsel (146) mit einem distalen Endabschnitt (142) des Schafts (134) verbunden ist und die drehbare Komponente (172) an einen proximalen Endabschnitt (140) des Schafts (134) wirkgekoppelt ist, so dass durch Drehung der drehbaren Komponente (172) eine axiale Bewegung des Schafts (134) und der Zuführkapsel (146) erzeugt wird.

8. Zuführvorrichtung (100) nach einem der Ansprüche 5 bis 7, wobei der Griffabschnitt (132) eine Öffnung (202) zum Einführen der Zugschnur (200) durch den Griffabschnitt (132) umfasst,
optional wobei die Zugschnur (200) dazu ausgestaltet ist, in einer im Wesentlichen senkrecht zu einer Längsachse des Griffabschnitts (132) verlaufenden Richtung durch die Öffnung (202) gezogen zu werden, um eine Drehung der drehbaren Komponente (172) zu erzeugen.

9. Zuführvorrichtung (100) nach Anspruch 8, wobei die drehbare Komponente (172) durch eine Bewegung der Zugschnur (200) in einer ersten Richtung durch die Öffnung (202) in eine zweite Richtung gedreht wird, um eine Bewegung der Zuführkapsel (146) in einer dritten, proximalen Richtung zu erzeugen, und die drehbare Komponente (172) durch eine Bewegung der Zugschnur (200) in einer vierten Richtung durch die Öffnung (202) in eine der zweiten Richtung entgegengesetzte fünfte Richtung gedreht wird, um eine Bewegung der Zuführkapsel (146) in einer sechsten, distalen Richtung zu erzeugen.

10. Zuführvorrichtung (100) nach einem der Ansprüche 5 bis 9, wobei der Motor (168) ein elektrisch betätigter Motor ist, der von mindestens einer Batterie mit Energie versorgt wird, die in dem Griffabschnitt (132) untergebracht ist.

11. Zuführvorrichtung (100) nach einem der Ansprüche 5 bis 10, wobei die Zugschnur (200) aus dem Griffabschnitt (132) entfernbar ist.

12. Zuführvorrichtung (100) nach einem der Ansprüche 5 bis 11, wobei die drehbare Komponente (172) eine Vielzahl von Zahnradzähnen (176) umfasst und wobei die Zugschnur (200) eine Vielzahl von Zähnen (204) umfasst, die dazu ausgestaltet sind, die Zahnradzähne (176) der drehbaren Komponente (172) in Antriebseingriff zu nehmen,
optional ferner umfassend ein Antriebszahnrad (182), das an den Motor (168) gekoppelt ist eine Vielzahl von Zähnen (192) umfasst, die die Zahnradzähne (176) der drehbaren Komponente (172) in Eingriff nehmen.

13. Zuführvorrichtung (100) nach Anspruch 12, wobei die Zugschnur (200) mindestens einen Endabschnitt (406) ohne Zähne umfasst.

14. Zuführvorrichtung (100) nach einem der Ansprüche 5 bis 13, wobei die Zugschnur (200) einen mittleren Abschnitt (404), der eine Vielzahl von Zähnen (408) umfasst, und zwei Endabschnitte (406) ohne Zähne umfasst, wobei der mittlere Abschnitt (404) zwischen den beiden Endabschnitten (406) angeordnet ist.

15. Zuführvorrichtung (100) nach einem der Ansprüche 5 bis 14, wobei die Zugschnur (200):
a) entlang ihrer Länge gerade ist oder
b) entlang mindestens eines Abschnitts ihrer Länge gewendelt ist und wobei ein gewendelter Abschnitt (504) der Zugschnur (200) eine Vielzahl von Zähnen (508) umfasst.

## Revendications

1. Appareil médical destiné à être inséré dans un sujet, l'appareil médical comprenant :
une partie poignée (132) ;
un composant mobile (146) configuré pour être inséré dans le sujet ;
un composant rotatif (172) disposé dans la partie poignée (132) et couplé de manière opérationnelle au composant mobile (146) pour produire un mouvement axial du composant mobile (146) par rapport à la partie poignée (132) lors de la rotation du composant rotatif (172) ;
un moteur (168) disposé dans la partie poignée (132) et couplé de manière opérationnelle au composant rotatif (172) pour produire une rotation du composant rotatif (172) et un mouvement axial correspondant du composant mobile (146) ; et
un cordon de traction (200) configuré pour produire une rotation du composant rotatif (172) et un mouvement axial correspondant du composant mobile (146) lorsqu'une force de traction manuelle est appliquée au cordon de traction (200) pour tirer le cordon de traction (200) par rapport au composant rotatif (172).

2. Appareil médical selon la revendication 1, le composant mobile (146) comprenant une capsule d'administration (146) configurée pour retenir un dispositif médical implantable (10) dans un état comprimé radialement pour l'administration dans le sujet.

3. Appareil médical selon la revendication 1 ou selon la revendication 2, la partie poignée (132) comprenant une ouverture (202) pour insérer le cordon de traction (200) à travers la partie poignée (132) et le cordon de traction (200) étant configuré pour être tiré à travers l'ouverture (202) dans une direction sensiblement perpendiculaire à un axe longitudinal de la partie poignée (132) pour produire une rotation du composant rotatif (172).

4. Appareil médical selon n'importe quelle revendication précédente, le composant rotatif (172) comprenant une pluralité de dents d'engrenage (176), le cordon de traction (200) comprenant une pluralité de dents (204) qui sont configurées pour venir en prise par entraînement avec les dents d'engrenage (176) du composant rotatif (172), et le cordon de traction (200) comprenant une partie centrale (404) comprenant la pluralité de dents (408) et deux parties d'extrémité (406) sans dents, la partie centrale (404) étant disposée entre les deux parties d'extrémité (406).

5. Appareil d'administration (100) pour un dispositif médical extensible et implantable (10), l'appareil d'administration (100) comprenant l'appareil médical selon la revendication 1 et comprenant en outre un arbre (134) s'étendant à partir de la partie poignée (132) ;
le composant mobile (146) comprenant une capsule d'administration (146) configurée pour loger le dispositif médical (10) dans un état radialement comprimé pour l'administration dans un sujet.

6. Appareil d'administration (100) selon la revendication 5, l'arbre (134) étant un premier arbre (134) et l'appareil d'administration (100) comprenant en outre un second arbre (150) s'étendant à travers le premier arbre (134), le second arbre (150) ayant une partie d'extrémité proximale (151) couplée de manière opérationnelle au composant rotatif (172) et une partie d'extrémité distale couplée de manière opérationnelle à la capsule d'administration (146) de telle sorte qu'une rotation du composant rotatif (172) fait tourner le second arbre (150) par rapport au premier arbre (134) et produit un mouvement axial de la capsule d'administration (146).

7. Appareil d'administration (100) selon la revendication 5, la capsule d'administration (146) étant reliée à une partie d'extrémité distale (142) de l'arbre (134) et le composant rotatif (172) étant couplé de manière opérationnelle à une partie d'extrémité proximale (140) de l'arbre (134) de telle sorte qu'une rotation du composant rotatif (172) produit un mouvement axial de l'arbre (134) et de la capsule d'administration (146).

8. Appareil d'administration (100) selon l'une quelconque des revendications 5 à 7, la partie poignée (132) comprenant une ouverture (202) permettant d'insérer le cordon de traction (200) à travers la partie poignée (132),
éventuellement, le cordon de traction (200) étant configuré pour être tiré à travers l'ouverture (202) dans une direction sensiblement perpendiculaire à un axe longitudinal de la partie poignée (132) pour produire une rotation de l'élément rotatif (172).

9. Appareil d'administration (100) selon la revendications 8, un mouvement du cordon de traction (200) à travers l'ouverture (202) dans une première direction faisant tourner le composant rotatif (172) dans une deuxième direction pour produire un mouvement de la capsule d'administration (146) dans une troisième direction proximale, et un mouvement du cordon de traction (200) à travers l'ouverture (202) dans une quatrième direction, opposée à la première direction, faisant tourner le composant rotatif (172) dans une cinquième direction, opposée à la deuxième direction pour produire un mouvement de la capsule d'administration (146) dans une sixième direction distale.

10. Appareil d'administration (100) selon l'une quelconque des revendications 5 à 9, le moteur (168) étant un moteur à actionnement électrique alimenté par au moins une batterie, qui est logée dans la partie poignée (132).

11. Appareil d'administration (100) selon l'une quelconque des revendications 5 à 10, le cordon de traction (200) étant amovible de la partie poignée (132).

12. Appareil d'administration (100) selon l'une quelconque des revendications 5 à 11, le composant rotatif (172) comprenant une pluralité de dents d'engrenage (176), et le cordon de traction (200) comprenant une pluralité de dents (204) qui sont configurées pour venir en prise par entraînement avec les dents d'engrenage (176) du composant rotatif (172),
comprenant éventuellement un engrenage d'entraînement (182) couplé au moteur (168) et comprenant une pluralité de dents (192) qui viennent en prise avec les dents d'engrenage (176) de l'élément rotatif (172).

13. Appareil d'administration (100) selon la revendication 12, le cordon de traction (200) comprenant au moins une partie d'extrémité (406) sans dents.

14. Appareil d'administration (100) selon l'une quelconque des revendications 5 à 13, le cordon de traction (200) comprenant une partie centrale (404) comprenant une pluralité de dents (408) et deux parties d'extrémité (406) sans dents, la partie centrale (404) étant disposée entre les deux parties d'extrémité (406).

15. Appareil d'administration (100) selon l'une quelconque des revendications 5 à 14, le cordon de traction (200) :
a) étant rectiligne sur toute sa longueur ; ou
b) étant enroulée sur au moins une partie de sa longueur, et une partie enroulée (504) du cordon de traction (200) comprenant une pluralité de dents (508).
